# EUROPEAN PATENT SPECIFICATION

(11) **EP 3 003 490 B1**
(45) Date of publication and mention of the grant of the patent: **13.11.2019**
(21) Application number: 14807920.5
(22) Date of filing: 03.06.2014
(51) Int. Cl.: A61P 35/00, A61K 36/074, A61K 31/337, A61K 36/537, A61K 36/539

(54) **COMBINATION THERAPY FOR PROSTATE CANCER USING BOTANICAL COMPOSITIONS AND DOCETAXEL**
KOMBINATIONSTHERAPIE FÜR PROSTATAKREBS MIT PFLANZLICHEN ZUSAMMENSETZUNGEN UND DOCETAXEL
POLYTHÉRAPIE PERMETTANT DE TRAITER UN CANCER DE LA PROSTATE AU MOYEN DE COMPOSITIONS BOTANIQUES ET DE DOCÉTAXEL

(30) Priority: 03.06.2013 US 201361830626 P
(43) Date of publication of application: 13.04.2016
(73) Proprietor: Genyous Biomed International, Redwood Shores, California 94065 (US)
(72) Inventor: DAO, James, Henderson, Nevada 89052 (US); DAO, Jeffery, San Mateo, California 94402 (US); GERWICK, William, La Jolla, California 92037 (US); GERWICK, Lena, La Jolla, California 92037 (US); JORDAN, MaryAnn, Santa Barbara, California 93105 (US); WILSON, Leslie, Carpinteria, California 93013 (US)
(74) Representative: J A Kemp LLP
(86) International application number: PCT/US2014/040769
(87) International publication number: WO 2014/197519

(56) References cited:
- WO-A1-2012/061790
- US-A1- 2002 156 125
- US-A1- 2005 196 409
- US-A1- 2013 101 616
- QU SIFENG ET AL: "Enhanced anticancer activity of a combination of docetaxel and Aneustat (OMN54) in a patient-derived, advanced prostate cancer tissue xenograft model", MOLECULAR ONCOLOGY, ELSEVIER, AMSTERDAM, NL, vol. 8, no. 2, 15 December 2013 (2013-12-15), pages 311-322, XP028661441, ISSN: 1574-7891, DOI: 10.1016/J.MOLONC.2013.12.004
- REBEKAH A. BURICH ET AL: "Genistein combined polysaccharide enhances activity of docetaxel, bicalutamide and Src kinase inhibition in androgen-dependent and independent prostate cancer cell lines", BJU INTERNATIONAL, 1 July 2008 (2008-07-01), pages ???-???, XP055113347, ISSN: 1464-4096, DOI: 10.1111/j.1464-410X.2008.07826.x
- MARCONETT, C.N. ET AL: 'BZL101, A PHYTOCHEMICAL EXTRACT FROM THE SCUTELLARIA BARBATA PLANT, DISRUPTS PROLIFERATION OF HUMAN BREAST AND PROSTATE CANCER CELLS THROUGH DISTINCT MECHANISMSDEPENDENT ON THE CANCER CELL PHENOTYPE' CANCER BIOL THER., [Online] vol. 10, no. 4, 15 August 2010, pages 397 - 405, XP055299744 DOI: 10.4161/CBT.10.4.12424 Retrieved from the Internet: <URL:HTTP://WWW.NCBI.NLM.NIH.GOV/PMC/ARTICL ES/PMC3040855/>
- ZHANG, Y. ET AL: 'TANSHINONES FROM CHINESE MEDICINAL HERB DANSHEN (SALVIA MILTIORRHIZA BUNGE) SUPPRESS PROSTATE CANCER GROWTH AND ANDROGEN RECEPTOR SIGNALING' PHARMACEUTICAL RESEARCH, [Online] vol. 29, no. 6, June 2012, pages 1595 - 1608, XP035053907 Retrieved from the Internet: <URL:HTTP://RD.SPRINGER.COM/ARTICLE/10.1007 %2FS11095-012-0670-3>
- MAHAJNA, J. ET AL: 'PHARMACOLOGICAL VALUES OF MEDICINAL MUSHROOMS FOR PROSTATE CANCER THERAPY: THE CASE OF GANODERMA LUCIDUM' NUTRITION AND CANCER, [Online] vol. 61, no. 1, 30 December 2008, pages 16 - 26, XP055299151 DOI: 10.1080/01635580802379323 Retrieved from the Internet: <URL:HTTP://WWW.TANDFONLINE.COM/DOI/ABS/10. 1080/01635580802379323>

## Description

### TECHNICAL FIELD OF THE INVENTION

This invention relates generally to the field of compositions for treatment of cancer. More specifically, the invention provides multifunctional, multitargeted compositions of botanical extracts in combination with docetaxel for the prevention and therapy of cancer, and specifically prostate cancer.

### BACKGROUND OF THE INVENTION

Prostate cancer is cancer that begins in tissues of the prostate gland. Located just below the bladder and in front of the rectum, the prostate is the male sex gland responsible for the production of semen. Other than skin cancer, prostate cancer is the most common cancer in American men. The American Cancer Society's estimates for prostate cancer in the United States for 2013 are: (a) about 238,590 new cases of prostate cancer will be diagnosed; (b) about 29,720 men will die of prostate cancer; (c) about 1 man in 6 will be diagnosed with prostate cancer during his lifetime.

Prostate cancer occurs mainly in older men. Nearly two thirds are diagnosed in men aged 65 or older, and it is rare before age 40. The average age at the time of diagnosis is about 67. Prostate cancer is the second leading cause of cancer death in American men, behind only lung cancer. About 1 man in 36 will die of prostate cancer. Prostate cancer can be a serious disease, but most men diagnosed with prostate cancer do not die from it. In fact, more than 2.5 million men in the United States who have been diagnosed with prostate cancer at some point are still alive today.

Fortunately, prostate cancer is one of the most treatable malignancies if it's caught early. Routine screening has improved the diagnosis of prostate cancer in recent years. The prostate is a walnut-sized organ that surrounds the urethra; it produces a fluid that becomes part of semen. More than 99 percent of prostate cancers develop in the gland cells. This type of prostate cancer is called adenocarcinoma. More rarely, prostate cancer originates in other tissues of the prostate, which is called a sarcoma.

Most prostate cancers are slow growing; however, there are cases of aggressive prostate cancers. The cancer cells may metastasize (spread) from the prostate to other parts of the body, particularly the bones and lymph nodes. Prostate cancer may cause pain, difficulty in urinating, problems during sexual intercourse, or erectile dysfunction. Other symptoms can potentially develop during later stages of the disease.

Treatment options for prostate cancer with intent to cure are primarily surgery, radiation therapy, and proton therapy. Other treatments, such as hormonal therapy, chemotherapy, cryosurgery, and high intensity focused ultrasound (HIFU) also exist, depending on the clinical scenario and desired outcome.

The age and underlying health of the man, the extent of metastasis, appearance under the microscope, and response of the cancer to initial treatment are important in determining the outcome of the disease. The decision whether or not to treat localized prostate cancer (a tumor that is contained within the prostate) with curative intent is a patient trade-off between the expected beneficial and harmful effects in terms of patient survival and quality of life.

Chemotherapy (chemo) is the treatment of cancer with drugs that destroy cancer cells and tumors. Prostate cancer chemotherapy is typically reserved for patients whose cancer has metastasized to the bone or elsewhere in the body. In some cases, chemo may be used in conjunction with other treatments, such as radiation, for the treatment of localized prostate cancer. In these cases, low-dose chemotherapy is used to sensitize the cancer cells to radiation therapy. Systemic chemotherapy can be taken by mouth or injected into a vein or muscle, and is designed to reach cancer cells throughout the body. Regional chemotherapy is delivered directly into the prostate or another part of the body to target cancer cells in specific areas.

Prostate cancer chemotherapy treatments are individualized for each patient. Blood counts and other health indicators are examined to choose the right combination of chemotherapy drugs, along with other prostate cancer treatments, if any. Until recently, the chemotherapies used were limited to cyclophosphamide, anthracyclines (doxorubicin or mitoxantrone) and estramustine, and the effects of these treatments are relatively mediocre. Palliative effects were observed in patients following the administration of corticoids alone or of mitoxantrone with either prednisone or hydrocortisone. Following Phase II trials, the combination of mitoxantrone with corticoids was recognized as the reference treatment for hormone-resistant prostate cancer.

Taxanes (such as paclitaxel and docetaxel) have been shown to have significant antineoplastic and anticancer effects in a wide variety of cancers. For example, paclitxel acts by interfering with the normal function of microtubule breakdown. Paclitaxel binds to the beta subunit of tubulin, the building blocks of microtubules, causing hyper-stabilization of the microtubule structures. The resulting paclitaxel/microtubule structure is unable to disassemble, thereby arresting mitosis and inhibiting angiogenesis. The poor aqueous solubility for the taxanes, however, presents significant challenges for developing effective taxane-based cancer therapeutics. Further, the interaction of different taxane formulations with other therapeutic agents in the combination therapy context remains to be studied.

Docetaxel is of the chemotherapy drug class; taxane, and is a semi-synthetic analogue of paclitaxel (Taxol), an extract from the bark of the rare Pacific yew tree Taxus brevifolia. Due to scarcity of paclitaxel, extensive research was carried out leading to the formulation of docetaxel - an esterified product of 10-deacetyl baccatin III, which is extracted from the renewable and readily available European yew tree. Docetaxel differs from paclitaxel at two positions in its chemical structure. It has a hydroxyl functional group on carbon 10, whereas paclitaxel has an acetate ester, and a tert-butyl carbamate ester exists on the phenylpropionate side chain instead of the benzyl amide in paclitaxel. The carbon 10 functional group change causes docetaxel to be more water soluble than paclitaxel. (Clarke SJ, Rivory LP (February 1999). "Clinical pharmacokinetics of docetaxel". Clin Pharmacokinet 36 (2): 99-114.)

Docetaxel is marketed worldwide under the name Taxotere® by Sanofi-Aventis. Docetaxel is used to treat advanced prostate cancer; specifically, in men whose prostate cancer has spread beyond the prostate and which has become resistant to hormone therapy. For men at this stage of cancer, there are not many treatment choices, as they have likely already undergone radiation therapy or surgery and have also tried and then developed resistance to hormone therapy. (Martel CL, Gumerlock PH, Meyers FJ, et al. Current strategies in the management of hormone refractory prostate cancer. Cancer Treat Rev. 2003; 29:171-187.) Docetaxel is administered via a one-hour infusion every three weeks over ten or more cycles.

Docetaxel, when used with or without prednisone, was the first chemotherapy drug proven to help patients with advanced prostate cancer live longer. (Tannock IF, de Wit R, Berry WR, et al. Docetaxel plus prednisone or mitoxantrone plus prednisone for advanced prostate cancer. N. Engl. J. Med. 351 (15): 1502-12 (2004)). Treatments with docetaxel in combination with estramustine or prednisone have made it possible to treat cancers that are resistant to hormone deprivation. The TAX 327 trial was a phase III study that showed significant survival benefit from docetaxel in androgen-independent metastatic prostate cancer. Compared with mitoxantrone treatment, docetaxel treated patients showed a 12% overall response rate and mitoxantrone showed a 7% overall response rate. Another large advantage of docetaxel was increased quality of life. (Berthold DR, Pond GR, Soban F, De Wit R, Eisenberger M, Tannock IF. Docetaxel plus prednisone or mitoxantrone plus prednisone for advanced prostate cancer: Updated survival in the TAX 327 study. J Clin Oncol. 2008; 26(2):242-245.)

Docetaxel is a chemotherapeutic agent and is a cytotoxic compound and so is effectively a biologically damaging drug. As with all chemotherapy, adverse effects are common and many varying side-effects have been documented. There is a need for enhanced cancer therapy regimens that increase effectiveness of the therapy while reducing side effects and toxicity resulting from the chemotherapeutic treatment.

Compositions of botanicals comprising therapeutically effective amounts of two or more of an extract of *Ganoderma lucidum,* an extract of *Salvia miltiorrhiza* and an extract of *Scutellaria barbata* for prevention and therapy of cancer have been reported by Dao et al. (US Pat.. No. 8,173,177).

### SUMMARY OF THE INVENTION

The present invention relates to combinations of docetaxel and compositions botanical extracts for treatment and therapy of prostate cancer.

In accordance with the present invention, there is provided a botanical composition for use in a method of treating or reducing the severity of prostate cancer in a subject, the method comprising:
administering an effective amount of the composition,
wherein the composition comprises extracts in an organic medium of *Ganoderma lucidum, Salvia miltiorrhiza* and *Scutellaria barbata,* wherein each extract comprises from about 45 percent to about 50 percent w/w of *Ganoderma lucidum* and *Scutellaria barbata* and about 1 to about 3 percent w/w of *Salvia miltiorrhiza*, wherein each extract is a non-alcoholic extract, and wherein the botanical composition is effective for reducing a symptom associated with prostate cancer; and
co-administering an effective amount of docetaxel,
wherein the co-administration of the compound and the botanical composition achieves more effective therapy than administration of either agent alone, and results in reduced toxicity and side-effects compared to administration of an equivalent dosage of docetaxel alone.

The compositions of botanical extracts can be used to reduce or alleviate the side affects when used with standard non-botanical chemotherapies. Side effects are reduced by inhibiting inflammatory responses, modulating immune responses, reducing oxidative stress, modulating immune responses, inhibiting viral and microbial infections, modulating cell proliferative responses or other biological responses. The compositions of the invention may also alleviate side affects of standard therapeutic agents by balancing general biological responses against perturbations in specific biological pathways due to treatment with the therapeutic agent.

The composition comprises combinations of extracts of *Ganoderma lucidum, Scutellaria barbata*, *Salvia miltiorrhiza*, and optionally, *Hippophae rhamnoides* (sea buckthorn).

We describe treatment or therapy of prostate cancer in a human, the method comprising: administering an effective amount of a botanical composition that is effective for reducing androgen receptor protein expression; and administering concurrently an effective amount of a compound having anti-androgen activity, wherein the concurrent administration of the compound and the botanical composition achieves more effective therapy than either agent alone. Botanical compositions as set forth in the claims comprising non-alcoholic organic extracts of *Ganoderma lucidum, Salvia miltiorrhiza* and *Scutellaria barbata* in conjunction with docetaxel therapy are used.

Methods are disclosed for treatment or reducing the severity of prostate cancer in a subject, the method comprising: administering an effective amount of a botanical composition comprising extracts in an organic medium of *Ganoderma lucidum, Salvia miltiorrhiza* and *Scutellaria barbata,* wherein each extract comprises from about 45 to about 50 percent w/w of *Ganoderma lucidum* and *Scutellaria barbata* and about 1 to about 3 percent w/w of *Salvia miltiorrhiza*, and wherein the botanical composition is effective for reducing a symptom associated with prostate cancer; and co-administering an effective amount of docetaxel, wherein the co-administration of the compound and the botanical composition achieves more effective therapy than administration of either agent alone.

In some aspects, the prostate cancer symptom is a size of a prostate tumor. In some aspects, the prostate tumor is an androgen-dependent adenocarcinoma. In some aspects, the prostate cancer symptom is a growth rate of prostate cancer tissue. In some aspects, the prostate cancer tissue is from an androgen-dependent adenocarcinoma. In some embodiments, the prostate cancer symptom is a level of prostate specific antigen (PSA).

In some embodiments, the co-adminstration comprises daily administration of the botanical composition and periodic administration of docetaxel over a time period. In some aspects, the time period is 3 weeks.

In some embodiments, the extract is a non-alcoholic organic extract. In some embodiments, the extract is made with ethyl acetate ester.

In some embodiments, co-administration of docetaxel and the botanical composition results in reduced toxicity and side-effects compared to administration of an equivalent dosage of docetaxel alone.

In some embodiments, the botanical composition comprises 2% w/w of *Salvia miltiorrhizza* extract.

In some embodiments, the botanical composition comprises one or more of an emulsifier, a surfactant, an antioxidant and a diluent. In some embodiments, the emulsifier is selected from one or more of fatty acids, polyoxyethylene glycerol esters of fatty acids, polooxylated castor oil, ethylene glycol esters, propylene glycol esters glyceryl esters of fatty acids, sorbitan esters, polyglyceryl esters, fatty alcohol ethoxylates, ethoxylated propoxylated block copolymers, polyethylene glycol esters of fatty acids, cremophores, glycerol monocaprylate/caprate, Cremophor EL, oleic acid, Labrasol, Gelucire, Capryol, Captex, Acconon, transcutol, and triacetin. In some embodiments, the antioxidant is selected from ascorbic acid and alpha tocopherol. In some embodiments, the diluent is soya oil.

In some embodiments, docetaxel is administered intra-venously. In some embodiments, the botanical composition is administered orally. In some embodiments, the botanical composition is in a dosage form suitable for oral administration.

In some aspects, the prostate cancer is a castration-resistant prostate cancer. In some aspects, the prostate cancer is an androgen-sensitive prostate cancer.

In some embodiments, the docetaxel is administered in a sub-therapeutic dose.

In some embodiments, the botanical composition comprises an effective amount of *Ganoderma lucidum* extract selected from 45%, 46%, 46.5%, 47%, 47.5%, 48%, 48.5%, 49%, 49.5% and 50%.

In some embodiments, the botanical composition comprises an effective amount of *Scutellaria barbata* extract selected from 45%, 46%, 46.5%, 47%, 47.5%, 48%, 48.5%, 49%, 49.5% and 50%.

In some embodiments, the botanical composition comprises an effective amount of *Salvia miltiorrhiza* extract selected from 1%, 1.5%, 2%, 2.5%, and 3%.

The present invention and other objects, features, and advantages of the present invention will become further apparent in the following Detailed Description of the Invention and the accompanying Figures and embodiments.

### BRIEF DESCRIPTION OF THE FIGURES

Figure 1 shows the effects of the combination of *Ganoderma lucidum, Scutellaria barbata*, and *Salvia miltiorrhiza* (Aneustat™, OMN54) and docetaxel treatment on growth of LNCaP (androgen-sensitive) human prostate cancer tumors SRCX in vivo (mice) (P<0.01).
Figure 2 shows the effects of the combination of *Ganoderma lucidum, Scutellaria barbata*, and *Salvia miltiorrhiza* (Aneustat™, OMN54) and docetaxel+estramustine treatment on growth of DU145 (androgen-independent) human prostate cancer tumors SRCX in vivo (mice) (P<0.01).
Figure 3 shows the synergistic effect of a combination of OMN54 and docetaxel on tumor shrinkage when treating LTL-313 (androgen-dependent) human prostate cancer tumors SRCX in vivo (mice) (p=0).
Figure 4 shows the synergistic effect of a combination of OMN54 and docetaxel on growth inhibition when treating LTL-313 (androgen-dependent; adenocarcinoma) patient-derived prostate cancer tissue SRCX. Dosages of TXT (Taxotere®), Combi-low and Combi-med are as described in Example 7.
Figure 5 shows the synergistic effect of a combination of OMN54 and docetaxel on PSA reduction-in a dose responsive manner-when treating LTL-313 (androgen-dependent; adenocarcinoma) patient-derived prostate cancer tissue SRCX.

### DETAILED DESCRIPTION OF THE INVENTION

### Definitions

The term "plant" as used herein refers to seeds, leaves, stems, flowers, roots, berries, bark, or any other plant parts that are useful for the purposes described. For certain uses, it is preferred that the underground portion of the plant, such as the root and rhizoma, be utilized. The leaves, stems, seeds, flowers, berries, bark, or other plant parts, also have medicinal effects and can be used for preparing tea and other beverages, cream, and in food preparation.

The term "treatment" or "treating" or 'therapy" as used herein, for purposes of the specification and claims, includes preventing, inhibiting, curing, or alleviating.

By the term "administering," it is meant that the compositions are delivered to the host in such a manner that it can achieve the desired purpose. As mentioned the compositions can be administered by an effective route, such as orally, topically, rectally, etc.

"Synergism" may be measured by combination index (CI). The combination index method was described by Chou and Talalay. (Chou, T.-C. The median-effect principle and the combination index for quantitation of synergism and antagonism, p. 61-102. In T.-C. Chou and D. C. Rideout (ed.), Synergism and antagonism in chemotherapy. Academic Press, San Diego, Calif. (1991); Chou, T.-C., and P. Talalay. Quantitative analysis of dose-effect relationships: the combined effects of multiple drugs on enzyme inhibitors. Adv. Enzyme Regul. 22:27-55 (1984)). A CI value of 0.90 or less is considered synergistic, with values of 0.85 being moderately synergistic and values below 0.75 being significantly synergistic. CI values of 0.90 to 1.10 are considered to be merely additive and higher values are antagonistic.

**Table 1. Synergism/antagonism as a function of CI values**

| **Combination Index (CI) Value** | **Interpretation** |
|---|---|
| >10 | Very strong antagonism |
| 3.3 - 10 | Strong antagonism |
| 1.45 - 3.3 | Antagonism |
| 1.2 - 1.45 | Moderate antagonism |
| 1.1 - 1.2 | Slight antagonism |
| 0.9 - 1.1 | Additive |
| 0.85 - 0.9 | Slight synergism |
| 0.7 - 0.85 | Moderate synergism |
| 0.3 - 0.7 | Synergism |
| 0.1 - 0.3 | Strong synergism |
| < 0.1 | Very strong synergism |

It is noted that determination of synergy may be affected by biological variability, dosage, experimental conditions (temperature, pH, oxygen tension, etc.), treatment schedule and combination ratio.

A botanical composition drug include: bio-availability and minimal toxicity. Preferably the drug can be administered orally. The botanical composition provides a combination of multiple therapeutic functions to act simultaneously and synergistically on multiple biological targets. The botanical composition comprises low doses of individual therapeutic ingredients to minimize disruption of physiological homeostasis and development of drug resistance. Typically, a history of therapeutic efficacy and safety is considered in selecting nature-derived active ingredients.

### Cancer:

Several cellular pathways have been implicated in cancer. Apoptotic pathway, mitogen-activated protein kinase (MAPK) Signaling Pathway, cell signaling via the phosphoinositide 3-kinase (PI3K) pathway, Signal transducers and activators of transcription (STAT) signaling pathway, p53 signaling pathway, *Wnt* signaling pathway, cyclooxygenase (COX) enzymes have all been known to contribute to several steps involved in tumor formation, such as neoplastic transformation, metastasis, and angiogenesis. Also, growth factors, oncogenes such as Ras mutations, tumor suppressor genes, androgen and estrogen receptors, co-activators & repressors, and numerous others pathways have been shown to affect tumor formation and carcinogenesis.

A botanical drug suitable for cancer would include functions to affect one or more of these pathways as well as functions generally related to alleviation of cancer conditions such as anti-inflammation, immune system modulation, anti-angiogenic, anti-metastatic, and the like.

A botanical drug suitable for a particular type of cancer will possess functionalities that also are directed to pathways unique to a type of cancer. For example, botanical drug suitable for the treatment of prostate cancer may include functionalities related to androgen receptors. Preferably, the botanical drug is administered in combination with standard chemotherapy regimens to achi.

Prostate cancer is a complex disease. A number of biological pathways have been implicated in prostate cancer development: growth factor activity, cell death (apoptosis), oncogenesis, tumor suppression, cell cycle modulation, cell surface modulation, androgen receptors, co-activators & repressors. Several conditions are associated with prostate disease: Benign prostate hyperplasia (BPH), prostatitis, prostatic intraepithelial neoplasia (PIN).

Benign prostatic hyperplasia (BPH) refers to the increase in size of the prostate in middle-aged and elderly men. BPH is characterized by hyperplasia of prostatic stromal and epithelial cells, resulting in the formation of large, fairly discrete nodules in the periurethral region of the prostate. Although prostate specific antigen levels may be elevated in these patients, because of increased organ volume and inflammation due to urinary tract infections, BPH is not considered to be a premalignant lesion.

Alpha blockers (α1-adrenergic receptor antagonists) provide symptomatic relief of BPH symptoms. When 5α-reductase inhibitors are used together with alpha blockers a reduction of BPH progression to acute urinary retention and surgery has been noted in patients with enlarged prostates. (Kaplan SA, McConnell JD, Roehrborn CG, et al (2006). Combination therapy with doxazosin and finasteride for benign prostatic hyperplasia in patients with lower urinary tract symptoms and a baseline total prostate volume of 25 ml or greater. J Urol 175(1): 217-20.)

Several botanicals show effectiveness in treating BPH and can be used as components of a botanical formulation. Serenoa repens (saw palmetto) fruit extracts are alleviating mild-to-moderate BPH symptoms with comparable efficacy to finasteride. (Wilt TJ, Ishani A, MacDonald R, (2002). Serenoa repens for benign prostatic hyperplasia. Cochrane Database Syst Rev 2002 (3), CD001423) Other botanicals effective in treating BPH include beta-sitosterol from Hypoxis rooperi (African star grass), pygeum (extracted from the bark of Prunus africana), Cucurbita pepo (pumpkin) seed and Urtica dioica (stinging nettle) root. (Wilt TJ, Ishani A, Rutks I, MacDonald R (2000) Phytotherapy for benign prostatic hyperplasia Public Health Nutr 3(4A):459-72). One double-blind trial has also supported the efficacy of rye flower pollen. (Buck AC, Cox R, Rees RWM, et al. (1990) Treatment of outflow tract obstruction due to benign prostatic hyperplasia with the pollen extract, Cernilton. A double-blind placebo-controlled study Br. J. Urol. 66:398-404).

Prostate cancer is classified as an adenocarcinoma, or glandular cancer, that begins when normal semen-secreting prostate gland cells mutate into cancer cells. Initially, small clumps of cancer cells remain confined to otherwise normal prostate glands, a condition known as carcinoma in situ or prostatic intraepithelial neoplasia (PIN). Although there is no clear evidence that PIN is a cancer precursor, it is closely associated with cancer.

Prostate specific antigen (PSA) is a 34 kD glycoprotein manufactured almost exclusively by the prostate gland. Also known as kallikrein III, PSA is a serine protease. (Lilja H. (Nov 2003). "Biology of Prostate-Specific Antigen". Urology 62 ((5 Suppl 1)): 27-33).

PSA is often elevated in the presence of prostate cancer and in other non-malignant prostate disorders such as BPH. A blood test to measure PSA is the most effective test currently available for the early detection of prostate cancer. Higher than normal levels of PSA are associated with both localized and metastatic prostate cancer (CaP). However, PSA levels can change for many reasons other than cancer. Two common causes of high PSA levels in the absence of cancer are enlargement of the prostate (benign prostatic hypertrophy (BPH)) and infection in the prostate (prostatitis).

Thus, PSA is not a perfect test. Some men with prostate cancer do not have an elevated PSA, and most men with an elevated PSA do not have prostate cancer. Short of biopsy, no non-invasive tests provide a clear diagnosis of prostate cancer.

### Botanical compositions for prostate cancer therapy

A botanical formulation presents an optimal first line therapy when high PSA levels are detected. Botanical compositions, such as those disclosed herein, have very low toxicity and yet are effective against prostate cancer.

The botanical composition was designed following demonstration of a number of desirable functions among the ingredients and in the assembled composition. A number of therapeutically active chemical entities are present in *Ganoderma lucidum* (#9), *Scutellaria barbata* (#15), and *Salvia miltiorrhiza (#14):* ganoderic acid A, crytotanshinone, tanshinone IIA, scutellarin tetramethyl ether, scutellarin, apigenin and wogonin.

A number of chemical entities are present in the botanical composition: adenosine, ganoderic acid A, oleic acid, tanshinone IIA, scutellarin, apigenin, luteolin, and wogonin. Each of these chemical entities is known to demonstrate one or more of anti-viral, anti-inflammatory, immune modulatory, anti-angiogenic and anti-cancer/metastatic functions.

An exemplary combination of *Ganoderma lucidum* (#9), *Scutellaria barbata* (#15), and *Salvia miltiorrhiza (#14)* was designated OMN54, based on the synergism displayed by certain combinations of extracts of the three botanicals when each botanical is between 1% w/w and 90% w/w of the combined composition. Extracts of the botanicals were preferably made in organic medium, such as alcohol, and non-alcoholic media including ester (such as ethyl acetate), lipid and the like. In a preferred embodiment the extracts were made in ethyl acetate medium.

Significant synergism was expressed by botanical compositions comprised of the following three organic extracts at the specified amounts (w/w):
(i) *Ganoderma lucidum* at 45 -50% w/w. More specifically the *Ganoderma lucidum* extract is selected from 45%, 46%, 46.5%, 47%, 47.5%, 48%, 48.5%, 49%, 49.5% and 50%.
(ii) *Scutellaria barbata* at 45 -50% w/w. More specifically the *Scutellaria barbata* extract is selected from 45%, 46%, 46.5%, 47%, 47.5%, 48%, 48.5%, 49%, 49.5% and 50%.
(iii) *Salvia miltiorrhiza* at 1-3% w/w. More specifically the *Salvia miltiorrhiza* extract is selected from 1%, 1.5%, 2%, 2.5%, and 3%.

In vitro studies have indicated that OMN54 did not modulate pro-inflammatory proteins in unstimulated PBMC (suggesting safety for long-term use). In the presence of inflammatory stimulus (PHA mitogen), OMN54 suppressed inflammatory signaling (significant anti-inflammatory activity). The effect of OMN54 in stimulated and unstimulated cells was observed for a number of proteins comprising cytokines, chemokines and growth factors.

The human prostate cancer cell line, LNCaP, is androgen sensitive and PSA positive. OMN54 was tested for activity on LNCaP cell line, on the expression of specific genes associated with prostate cancer. High levels of Prostate Specific Antigen (PSA) are associated with prostate cancer. The significant suppression of the PSA transcript by OMN54 is considered beneficial in the treatment of prostate cancer.

OMN54 displays profound antiproliferative effect on prostate cancer cells, inducing the apoptosis of both androgen receptor (AR)-positive (LNCaP) and AR-negative (DU-145) prostate cancer cell lines. OMN54 also displays NF-kappa B inhibition. The anti-inflammatory and anti-proliferative functions displayed by the botanical composition also are effective against BPH. Thus, following detection of high PSA levels and prior to invasive tests for diagnosis of cancer, a low toxicity botanical formulation which is effective against both BPH and neoplastic states like prostate cancer and PIN, provides a promising regimen for first intervention.

Other botanicals effective against prostate cancer include capsaicin, found in red peppers, which has a profound anti-proliferative effect on human prostate cancer cells in culture and in mouse xenografts. (Mori A et al. Cancer Res. 2006 Mar 15;66(6):3222-9). Capsaicin down-regulates PSA expression by direct inhibition of PSA transcription mediated by inhibition of NF-kappa B activation by preventing its nuclear migration. (*Id*.)

A 2006 study of green tea derivatives demonstrated prostate cancer prevention in patients at high risk for the disease. (Bettuzzi S, Brausi M, Rizzi F, Castagnetti G, Peracchia G, Corti A (2006). "Chemoprevention of human prostate cancer by oral administration of green tea catechins in volunteers with high-grade prostate intraepithelial neoplasia: a preliminary report from a one-year proof-of-principle study". Cancer Res 66 (2): 1234-40). A phytochemical called di-indolyl-methane found in cruciferous vegetables is suspected of having anti-androgenic and immune modulating properties.

Botanicals are a valuable resource for the discovery and development of novel, naturally derived agents to treat human disease. Botanical extracts usually comprise multiple molecules and possess multiple functions useful in the treatment and prevention of disease. Botanical extracts also can function to maintain normal tissue homeostasis by affecting multiple biological pathways such as the inflammatory pathway, the immune response pathway and the oxidative stress response pathway. As a result, botanical extracts can alleviate the harmful side effects of many therapeutic agents used to treat multiple disease targets.

Botanicals have been demonstrated to be a successful source of anticancer compositions. Examples include *Gynostemma pentaphyllum* extract, *Camellia sinensis* (green tea) and *Crataegus pinnatifida* (hawthorn berries) and a method of making the same are the subject of U.S. Pat. Nos. 5,910,308 and 6,168,795. Some drugs, derived from plants that are currently used in cancer therapy were designed to perturb microtubule shortening (depolymerization) or lengthening (polymerization), such as paclitaxel, docetaxel, etoposide, vincristine, vinblastine, and vinorelbine (Compton, D. A., et al., (1999) Science 286:913-914). They share a common mechanism of action of binding to tubulin, the molecule of which microtubules are composed. (Compton, D. A., et al., (1999) Science 286:913-914). At least six plant-derived anticancer agents have received FDA approval (e.g., taxol, vinblastine, vincristine, topotecan, etoposide, teniposide). Other agents are being evaluated in clinical trials (e.g., camptothecin, 9AC, and irinotecan). Botanical extracts for the treatment cancer are described in U. S. Pat. Application Publications 20050214394 A1, 20050208070 A1 and 20050196409 A1.

The present disclosure provides compositions comprising botanical extracts to treat human diseases that are associated with multiple biological pathways in their pathologies. The compositions of the disclosure are comprised of three or more botanical extracts which work synergistically to modulate multiple biological pathways including but not limited to inflammatory responses, immune responses, oxidative responses, viral and microbial infections, and cell proliferative responses.
(i) *Ganoderma lucidum* (Reishi): *Ganoderma lucidum* was praised for its effect of increasing memory and preventing forgetfulness in old age reported in Shen Nong Ben Cao Jing vol. 1 as early as 456-536 AD. Research on mice using orally or topically administered *Ganoderma lucidum* suggests that *Ganoderma lucidum* has anti-inflammatory activity. (Stavinoha, W., et al., (1995). Study of the anti-inflammatory efficacy of Ganoderma lucidum. In B.-K. Kim, & Y.S. Kim (Eds.), Recent Advances in Ganoderma lucidum research (pp. 3-7). Seoul Korea: The Pharmaceutical Society of Korea).
   Applications of *Ganoderma* for (1) chemoprophylaxis of cancer in individuals at high risk for developing cancer (2) adjuvant use in the prevention of metastasis or recurrence of cancer (3) palliation of cancer related cachexia and pain and (4) adjunctive use with concurrent chemotherapy to reduce side-effects, maintain leukocyte counts and allow a more optimal dosing of chemo or radio therapeutics has been suggested (Chang, R. (1994) Effective Dose of Ganoderma in Humans; Proceedings of Contributed Symposium 59A, B 5th International Mycological Congress, Vancouver: pp. 117-121). Since studies of human dosage were traditional and empirical, a proper dose range of *Ganoderma* for therapy was calculated using this data and pharmacokinetic principals. The calculations suggested that a (1) *Ganoderma* dried fruit body dose of 0.5 to 1 g per day for health maintenance (2) 2 to 5 g per day if there is chronic fatigue, stress, auto immune, or other chronic health problems (3) 5 to 10 g per day for serious illness. (Chang, R. (1993) Limitations and Potential applications of Ganoderma and related fungal polyglycans in clinical ontology; First International Conference on Mushroom Biology and Mushroom products: 96).
(ii) *Scutellaria barbata* (Skullcap): *Scutellaria barbata*, a traditional Chinese medicine for liver, lung and rectal tumors, has been shown to inhibit mutagenesis, DNA binding and metabolism of aflatoxin B1 (AFB1) and cytochrome P450-linked aminopyrine N-demethylase (Wong B.Y. et al., (1993) Eur. J. Cancer Prev. 2(4):351-6; Wong B.Y. et al., (1992) Mutat. Res. 279(3):209-16). *Scutellaria barbata* is also capable of enhancing macrophage function in vitro and inhibiting tumor growth in vivo (Wong B.Y. et al., (1996) Cancer Biother. Radiopharm. 11(1):51-6).
   This herb contains vitamins C and E as well as calcium, potassium, magnesium, iron, zinc scutellarin, volatile oil, tannin and bitter principles. The scutellarin acts on the central nervous system. Scutellarin, an active ingredient from *Scutellaria barbata* has been purified by liquid chromatography (Wenzhu Zhang et al., (2003) J. of Liquid Chromatography & Related Technologies 26 (13):2133-40).
(iii) *Scutellaria baicalensis: Scutellaria baicalensis* has been shown to have anti-proliferative and apoptotic activities against lymphocytic leukemia, lymphoma, and myeloma cell lines and possess anti-cancer activity on human malignant brain tumor cells (Kumagai, T. et al. (2006) Leuk. Res.; Scheck, A.C. et al., (2006) BMC Complement Altern. Med. 6:27).
   *Scutellaria barbata* should not be confused with *Scutellaria baicalensis. Banzhilian*, the whole plant of *Scutellaria barbata*, should not be confused with "scute," the common name referring to huangqin, the root of *Scutellaria baicalensis*."Although both are of the same genus, *Scutellaria barbata*, for which the tops are used, has essential oils among the active components, while *Scutellaria baicalensis* relies primarily on flavonoids, particularly baicalin and baicalein. Scutellaria radix (root of *Scutellaria baicalensis*) and *Scutellaria barbata* comprise different sets of flavonoids and show different effects on proliferation of human leukemia cell line HL-60. Sonoda et al., J. Ethnopharm 91:65-68 (2004).
(iv) *Salvia miltiorrhiza* (Dan Shen): There are over 900 species of salvia and many of them have histories of medicinal uses. Dan shen is used in traditional Chinese medicine to promote blood circulation and to remove blood stasis (Bensky D., Gamble A Chinese herbal Medicine Materia Medica 1987 Eastland Press: Seattle. 384). It increases the activity of SOD in platelets, thus providing protection against pulmonary embolism and inhibition of platelet aggregation. (Wang, X. et al., (1996) Zhongguo Zhong Yao Za Zhi 21:558-60). *Salvia miltiorrhiza* has been shown to lower cholesterol, reduce endothelial damage and to inhibit lipid peroxidation in hypercholesterolemic animals. This inhibition of oxidation of LDL may reduce atherosclerosis (Wu Y.J. et al., (1998) Arteriosclerosis Thromb Vasc Biol 18:481-6). A *Salvia miltiorrhiza* constituent has been found to inhibit noradrenalin-induced contraction of the aortic strips through reduction in Ca²⁺ mobilization. This vasodilatory activity may explain the traditional use of *Salvia miltiorrhiza* in hypertension (Nagai M. et al., Biol Pharm Bull (1996) 19:228-32). *Salvia miltiorrhiza* has been shown to have a markedly superior effect to nitroglycerin, with a more persistent action and better improvement of cardiac function (Bai, Y.R. and Wang, S.Z., (1994) Zhongguo Zhong Xi Yi Jie He Za Zhi 14:24-5, 4).
   *Salvia miltiorrhiza* is also the top ingredient in Dan Shen Compound. Dan Shen Compound comprises four important herbs for the improvement of peripheral circulation and general wellbeing. The actions of Crataegus levigata are enhanced by the Chinese herb *Salvia miltiorrhiza* (Dan Shen), the Indian herb Coleus forskohlii and Valeriana officinalis. Chinese herbal medicine utilizes *Salvia miltiorrhiza* for women's irregularities, abdominal pain, insomnia, hives, hepatitis and mastitis.
(v) *Hippophae rhamnoides* (sea buckthorn): Sea buckthorn seed oil contains a high content of the two essential fatty acids, linoleic acid and α-linolenic acid, which are precursors of other polyunsaturated fatty acids such as arachidonic and eicosapentaenoic acids. The oil from the pulp/peel of seabuckthorn berries is rich in palmitoleic acid and oleic acid (Chen et al., "Chemical composition and characteristics of seabuckthorn fruit and its oil." Chem. Ind. Forest Prod. (Chinese) 10 (3), 163-175). The increase in the level of α-linolenic acid in plasma lipids showed a clear improving effect on AD symptoms (Yang et al., (2000) J. Nutr Biochem. 11(6):338-340). These effects of α-linolenic acid may have been due to both changes in the eicosanoid composition and other mechanisms independent of eicosanoid synthesis (Kelley (1992) Nutrition, 8 (3), 215-2).
   Antioxidant and immunomodulatory properties of sea buckthorn (*Hippophae rhamnoides*) have been demonstrated using lymphocytes as a model system. (Geetha et al. J Ethnopharmacol 2002 Mar; 79(3):373-8). The antiulcerogenic effect of a hexane extract from *Hippophae rhamnoides* has also been demonstrated. (Suleyman H. et al., (2001) Phytother Res 15(7):625-7). Radioprotection by an herbal preparation of *Hippophae rhamnoides* against whole body lethal irradiation in mice suggests free radical scavenging, acceleration of stem cell proliferation and immunostimulation properties. (Goel H.C. et al., (2002) Phytomedicine 9(1):15-25).
(vi) *Camellia sinensis* (Green tea): Dried leaves from the *Camellia sinensis* plant is processed into three types of tea: oolong tea, black tea, and green tea. Green tea extract is a bioflavonoid-rich, potent extract which is used primarily for fighting free radicals. It has a high content of polyphenols, which are a type of bioflavonoids. In making green tea, the tea leaves are stabilized by moist or dry heat which destroys the enzyme polyphenoloxidase and thus, prevents oxidation of polyphenols. These polyphenols are the main biologically active ingredients in green tea. In preferred embodiments, the green tea is Dragon Well tea or Lung Ching tea.

The polyphenols in green tea are catechins, with multiple linked ring-like structures. Polyphenols are a form of bioflavonoids with several phenol groups. They control both taste and biological action. Catechins, a chemical group of polyphenols possessing antioxidant properties (protecting cells from free radical-mediated damage), include epigallocatechin-3 gallate (EGCG), epigallocatechin, and epicatechin-3-gallate. Recently, ECGC has been shown to be an inhibitor of urokinase (Jankun et al., (1997) Nature 387:561), and quinol-oxidase; enzymes that may be crucial for growth of tumor cells. Epigallocatechin-3 gallate (EGCG) also protects against digestive and respiratory infections.

Novel tumor inhibiting, immune boosting, inflammation reducing and anti-oxidative properties observed for compositions comprising a combination of three or more extracts of *Ganoderma lucidum, Scutellaria barbata*, *Scutellaria baicalensis,* and *Salvia miltiorrhiza* and, optionally, *Hippophae rhamnoides* (seabuckthorn) and *Camellia sinensis* (green tea) and the synergistic effects demonstrated by the present examples are a likely result of combinations of one or more of saponins, flavonoids, and polyphenols present in the extracts.

### Formulations of Botanical Compositions

The compositions of the present invention can be in any form which is effective, including, but not limited to dry powders, grounds, emulsions, extracts, and other conventional compositions. To extract or concentrate the effective ingredients of the compositions, typically the botanical part is contacted with a suitable solvent, such as water, alcohol, methanol, mixed solvents, or any other solvents. The choice of the solvent can be made routinely, e.g., based on the properties of the active ingredient that is to be extracted or concentrated by the solvent. Preferred active ingredients of the compositions crenulata include, but are not limited to, salidroside, tyrosol, β-sitosterol, gallic acid, pyrogallol, crenulatin, rhodionin, and/or rhodiosin. These ingredients can be extracted in the same step, e.g., using an alcoholic solvent, or they may be extracted individually, each time using a solvent which is especially effective for extracting the particular target ingredient from the plant. In certain embodiments, extraction can be performed by the following process: Milling the selected part, preferably root, to powder. The powder can be soaked in a desired solvent for an amount of time effective to extract the active agents from the compositions. The solution can be filtered and concentrated to produce a paste that contains a high concentration of the constituents extracted by the solvent. In some cases, the paste can be dried to produce a powder extract of the compositions crenulata. The content of active ingredient in the extract can be measured using HPLC, UV and other spectrometry methods.

The compositions of the present invention can be administered in any form by any effective route, including, e.g., oral, parenteral, enteral, intraperitoneal, topical, transdermal (e.g., using any standard patch), ophthalmic, nasally, local, non-oral, such as aerosol, inhalation, subcutaneous, intramuscular, buccal, sublingual, rectal, vaginal, intra-arterial, and intrathecal, etc. It can be administered alone, or in combination with any ingredient(s), active or inactive, including in a medicinal form, or as a food or beverage additive.

In preferred embodiments of the invention, the compositions are administered orally in any suitable form, including, e.g., whole plant, powdered or pulverized plant material, extract, pill, capsule, granule, tablet or a suspension.

The compositions can be combined with any pharmaceutically acceptable carrier. By the phrase, "pharmaceutically acceptable carriers," it is meant any pharmaceutical carrier, such as the standard carriers described, e.g., Remington's Pharmaceutical Science, 18th Edition, Mack Publishing company, 1990. Examples of suitable carriers are well known in the art and can include, but are not limited to, any of the standard pharmaceutical carriers such as a phosphate buffered saline solutions, phosphate buffered saline containing Polysorb 80, water, emulsions such as oil/water emulsion and various types of wetting agents. Other carriers may also include sterile solutions, tablets, coated tablets pharmaceutical and capsules. Typically such carriers contain excipients such as such as starch, milk, sugar, certain types of clay, gelatin, stearic acid or salts thereof, magnesium or calcium stearate, talc, vegetable fats or oils, gums, glycols. Such carriers can also include flavor and color additives or other ingredients. Compositions comprising such carriers are formulated by well known conventional methods. Generally excipients formulated with the compositions are suitable for oral administration and do not deleteriously react with it, or other active components.

Suitable pharmaceutically acceptable carriers include but are not limited to water, salt solutions, alcohols, gum arabic, vegetable oils, benzyl alcohols, gelatin, carbohydrates such as lactose, amylose or starch, magnesium stearate, talc, silicic acid, viscous paraffin, perfume oil, fatty acid monoglycerides and diglycerides, pentaerythritol fatty acid esters, hydroxy methylcellulose and the like. Other additives include, e.g., antioxidants and preservatives, coloring, flavoring and diluting agents, emulsifying and suspending agents, such as acacia, agar, alginic acid, sodium alginate, bentonite, carbomer, carrageenan, carboxymethylcellulose, cellulose, cholesterol, gelatin, hydroxyethyl cellulose, hydroxypropyl cellulose, hydroxypropyl methylcellulose, methylcellulose, octoxynol 9, oleyl alcohol, povidone, propylene glycol monostearate, sodium lauryl sulfate, sorbitan esters, stearyl alcohol, tragacanth, xanthan gum, and derivatives thereof, solvents, and miscellaneous ingredients such as microcrystalline cellulose, citric acid, dextrin, dextrose, liquid glucose, lactic acid, lactose, magnesium chloride, potassium metaphosphate, starch, and the like.

The botanical compositions can also be formulated with other active ingredients, such as anti-oxidants, vitamins (A, C, ascorbic acid, B's, such as B1, thiamine, B6, pyridoxine, B complex, biotin, choline, nicotinic acid, pantothenic acid, B12, cyanocobalamin, and/or B2, D, D2, D3, calciferol, E, such as tocopherol, riboflavin, K, K1, K2). Preferred compounds, include, e.g. creatine monohydrate, pyruvate, L-Carnitine, α-lipoic acid, Phytin or Phytic acid, Co Enzyme Q10, NADH, NAD, D-ribose, amino acids such as L-glutamine, Lysine, chrysin; prehormones such as 4-androstenedione, 5-androstenedione, 4(or 5-)-androstenediol, 19-nor-4(or 5-)-androstenedione, 19-nor-4 (or 5-)-androstenediol, Beta-ecdysterone, and 5-Methyl-7-Methoxy Isoflavone. Preferred active ingredients include, e.g., pine pollen, fructus lycii, *Hippophae rhamnoides*, Ligusticum, Acanthopanax, Astragalus, Ephedra, codonopsis, polygola tenuifolia Willd, Lilium, Sparganium, ginseng, panax notogiseng, Garcinia, Guggle, Grape Seed Extract or powder, and/or Ginkgo Biloba.

Other plants and herbs which can be formulated with the compositions of the present invention includes those mentioned in various text and publications, e.g., E.S. Ayensu, Medicinal Plants of West Africa, Reference Publications, Algonac, Mich. (1978); L. Boulos, Medicinal Plants of North Africa, Reference Publications Inc., Algonac, Mich. (1983); and N. C. Shah, (1982) J. Ethnopharm, 6:294-5.

A botanical formulation may comprise biologics and chemical entities, in addition to or in the place of, botanical extracts. Examples of biologics that may comprise a botanical composition include but are not limited to blood and blood products, cells, tissues and organs, gene therapy vectors, viral and bacterial vaccines, therapeutic products produced through biotechnology such as antibodies, monoclonal antibodies, and the like.

Pharmaceutically active agents that can comprise a botanical composition include, but are not limited to antioxidants, anticarcinogens, anti-inflammatory agents, hormones and hormone antagonists, anti-hypertensive agents, anti-inflammatory agents, tranquilizers, cardiotonic agents, antidepressants, corticosteroids, anti-ulcer agents, anti-allergy agents and anti-obesity agents, antibiotics, antibacterial agents, bacterial agents, and other medically useful drugs such as those identified in, e.g., Remington's Pharmaceutical Sciences, 18th Edition, Mack Publishing Company, 1990. A preferred composition of the present invention comprises, about 1%-100%, preferably about 20-70% of the botanical extract and, optionally, a pharmaceutically-acceptable excipient. Another preferred composition of the present invention comprises, about 1%-99%, preferably about 20-70% of botanical extracts, 0.1-99%, preferably 1-10% of one or more pharmaceutically active agents and, optionally, a pharmaceutically-acceptable excipient.

In some embodiments, the botanical composition comprises a chemotherapeutic agent either in a single formulation or separately administered as part of a therapeutic regimen.

### Docetaxel

The cytotoxic activity of docetaxel is exerted by promoting and stabilizing microtubule assembly, while preventing physiological microtubule depolymerisation/disassembly in the absence of GTP. This leads to a significant decrease in free tubulin, needed for microtubule formation and results in inhibition of mitotic cell division between metaphase and anaphase, preventing further cancer cell progeny. Because microtubules do not disassemble in the presence of docetaxel, they accumulate inside the cell and cause initiation of apoptosis. Apoptosis is also encouraged by the blocking of apoptosis-blocking bcl-2 oncoprotein. (Lyseng-Williamson KA, Fenton C (2005). "Docetaxel: a review of its use in metastatic breast cancer". Drugs 65 (17): 2513-31). Docetaxel is used in a number of cancer therapeutic regimens:

Breast Cancer: TAXOTERE® is indicated for the treatment of patients with locally advanced or metastatic breast cancer after failure of prior chemotherapy TAXOTERE® in combination with doxorubicin and cyclophosphamide is indicated for the adjuvant treatment of patients with operable node-positive breast cancer.

Advanced Non-Small Cell Lung Cancer: TAXOTERE®, as a single agent, is indicated for the treatment of patients with locally advanced or metastatic non-small cell lung cancer (NSCLC) after failure of prior platinum-based chemotherapy TAXOTERE® in combination with cisplatin is indicated for the treatment of patients with unresectable, locally advanced or metastatic NSCLC who have not previously received chemotherapy for this condition.

Advanced Gastric/GE Junction Cancer: TAXOTERE® in combination with cisplatin and fluorouracil is indicated for the treatment of patients with advanced gastric adenocarcinoma, including adenocarcinoma of the gastroesophageal junction, who have not received prior chemotherapy for advanced disease.

Locally Advanced Head and Neck Cancer: TAXOTERE® in combination with cisplatin and fluorouracil is indicated for the induction treatment of patients with locally advanced squamous cell carcinoma of the head and neck (SCCHN).

Metastatic Androgen-Independent Prostate Cancer: TAXOTERE® in combination with prednisone is indicated for the treatment of patients with androgen-independent (hormone-refractory) metastatic prostate cancer. Taxotere® 75 mg/m² IV infusion over 1 hour, administered every 3 weeks. Prednisone 5 mg orally twice daily is administered continuously. Taxotere® in combination with prednisone should be administered when the neutrophil count is ≥1500 cells/mm³.

According to the instant invention, it has been surprisingly observed that concurrent treatment with Docetaxel and OMN54 results in more effective remediation of prostate cancer symptoms with significantly reduced side effects and synergistic anticancer activity.

For treating Metastatic Androgen-Independent Prostate Cancer TAXOTERE® is administered by 75 mg/m² IV infusion over 1 hour, administered every 3 weeks. Prednisone 5 mg orally twice daily is administered continuously. Taxotere® in combination with prednisone should be administered when the neutrophil count is ≥1500 cells/mm³.

The instant invention relates to the administration of docetaxel and the botanical compositions (such as OMN54) concurrently. By concurrently, it is understood that the botanical composition is administered at about the same time, or the same day, or within 24 hours of docetaxel. The typical concentration of OMN54 is 25, 50, 100, 200 or 500 µg/ml and docetaxel is at a concentration of 1 nM.

In some embodiments, the botanical composition is formulated as an oral dosage form comprising a composition comprising the extracts of *Ganoderma lucidum, Scutellaria barbata*, and *Salvia miltiorrhiza*, and optionally other pharmaceutically acceptable excipients. In some embodiments, the dosage form comprises a bilayered dosage form. In some aspects the tablet is coated with one or more enteric polymers, pharmaceutically acceptable seal coat polymers or rate controlling polymers. In some aspects, the active ingredients are provided as a dispersion provided in a capsule, granule, mini-tablet or tablet form.

Docetaxel when used as Taxotere® is administered intravenously by 1 hour infusions. Each vial of Taxotere® Injection Concentrate is a sterile, non-pyrogenic, pale yellow to brownish-yellow solution at 20 mg/mL concentration. Each mL of 1-vial Taxotere® contains 20 mg docetaxel (anhydrous) in 0.54 grams polysorbate 80 and 0.395 grams dehydrated alcohol solution. (Taxotere® Prescribing Information. Bridgewater, NJ: Sanofi-Aventis U.S. LLC; September 2011.) A three-week administration schedule used to be and is still considered effective but new studies are indicating a weekly schedule might be better.

The present invention relates to methods of administering the compositions, e.g., to provide anti-inflammatory effects, to reduce inflammation, to provide antioxidant effects, to protect against oxidation, to provide antiproliferative effects, to provide anti-cancer effects, to promote DNA repair, to provide anti-radiation effects, to protect against radiation, and other conditions and diseases as mentioned herein.

An effective amount of the compositions are administered to such a host. Effective amounts are such amounts which are useful to achieve the desired effect, preferably a beneficial or therapeutic effect as described above. Such amount can be determined routinely, e.g., by performing a dose-response experiment in which varying doses are administered to cells, tissues, animal models (such as rats or mice in maze-testing, swimming tests, toxicity tests, memory tests as performed by standard psychological testing, etc.) to determine an effective amount in achieving an effect. Amounts are selected based on various factors, including the milieu to which the composition is administered (e.g., a patient with cancer, animal model, tissue culture cells, etc.), the site of the cells to be treated, the age, health, gender, and weight of a patient or animal to be treated, etc. Useful amounts include, 10 milligrams-100 grams, preferably, e.g., 100 milligrams-10 grams, 250 milligrams-2.5 grams, 1 gm, 2 gm, 3 gm, 500 milligrams-1.25 grams. etc., per dosage of different forms of the compositions such as the botanical powder, botanical extract paste or powder, tea and beverages prepared to contain the effective ingredients of the compositions, and injections, depending upon the need of the recipients and the method of preparation.

The liquid, pharmaceutically active formulation comprises a pharmaceutically active botanical composition in a liquid diluent or carrier. The active ingredient may be dissolved or dispersed in the liquid diluent or carrier, which may be a water miscible or water immiscible medium. Examples of liquid diluents or carriers include the following three classes: (a) Water miscible carriers: Propylene Glycol, Polyethylene Glycol, Water, Solketal, Glycofurol, Dimethylisosorbide, Nonionic surface active agents; (b) Oils and Organic carriers: Fractionated Coconut Oil, Sesame Oil, Soya Bean Oil, Vegetable Oil, Liquid Paraffin, Isopropylmyristate, Triacetin; and (c) Semi-solid carriers: High molecular weight polyethylene glycols, and White soft paraffin.

In some embodiments, one or more emulsifiers or surfactants are included in the formulation. Suitable emulsifiers which can be used include one or more of fatty acids such as oleic acid, polyoxyethylene glycerol esters of fatty acids, such as Tagats; polooxylated castor oil, ethylene glycol esters, such as glycol stearate and distearate; propylene glycol esters, such as propylene glycol myristate; glyceryl esters of fatty acids, such as glyceryl stearates and monostearates; sorbitan esters, such as spans and tweens; polyglyceryl esters, such as polyglyceryl 4-oleate; fatty alcohol ethoxylates, such as Brij type emulsifiers; ethoxylated propoxylated block copolymers, such as poloxamers; polyethylene glycol esters of fatty acids, such as Labrafils, Labrafacs, and Labrasols; cremophores; glycerol monocaprylate/caprate, such as Campmul CM 10; Gelucire, Capryol, Captex, Acconon, transcutol, triacetin, and the like. In some embodiments, the emulsifying agent is selected from Cremophor EL, oleic acid and labrasol. In some embodiments, antioxidants and/or diluents are used in the formulation. In some embodiments, antioxidants are selected from selected from ascorbic acid and alpha tocopherol. In some embodiments, the diluent is soyabean oil.

Compositions of the present invention comprise effective amounts of a combination of extracts of *Ganoderma lucidum, Scutellaria barbata*, and, *Salvia miltiorrhiza*, and optionally, *Hippophae rhamnoides* (sea buckthorn) that exhibit synergy in an ant-proliferation or anti-inflammation assay.

In one aspect of the invention, the botanical composition comprises effective amounts of extracts of *Ganoderma lucidum, Scutellaria barbata*, and *Salvia miltiorrhiza*. The dosage of the composition can be readily determined by one of skill in the art based on the effective concentrations of compositions shown to display the various properties described herein.

Compositions comprising different ratios of the individual extracts can similarly be determined. For example, a composition may exhibit anti-inflammatory effects at one concentration or ratios of combinations of extracts and varying degrees of cytotoxic effects at other concentrations or ratios of combinations of extracts. OMN54 comprises about 1-3% *Salvia miltiorrhiza,* and approximately equal amounts (45-50%) of *Scutellaria barbata* and *Ganoderma lucidum.*

In a further aspect, the compositions of the present disclosure comprise botanical compounds that are useful in compositions to be administered in conjunction with therapeutic agents for the treatment of disease. These compositions exhibit synergistic action with the therapeutic agent based on their anti-inflammatory, antioxidant, immune modulating, antiviral, antibacterial, antiproliferative activity or any combination of activities thereof.

The compositions demonstrate antioxidant activity which prevents damage to chromosomes/genes, reduces effect of mutagens, alleviates side-effects of chemotherapeutic agents, alleviates side-effects of hormone therapeutic agents, and enhances cell repair mechanisms.

The compositions further demonstrate immune system boosting activity which facilitates elimination of (i) damaged cells or (ii) cells with damaged genes. Further, the compositions provide general benefits of improving immune condition (passive immunotherapy).

The botanical sources of the extracts are botanicals that are essentially nontoxic with a long history of usage of the individual compounds/extracts. Anti-mutagenic properties as evidenced by Ames test results (together with increased sensitivity by synergism) reduce levels of chemotherapeutic agents necessary for treatment resulting in reduced toxicity for patients.

The botanical compositions demonstrate the ability to enhanced cell cycling which could make the botanical composition of the invention a powerful adjuvant to chemotherapy (e.g., with docetaxel), hormonal therapy, or radiation therapy by increasing effectiveness and reducing necessary dosages of chemotherapeutic agents and hormone therapeutic agents.

Quality control. IC₅₀ based compositions can be standardized based on specific activities of defined properties.

The botanical compositions are also suited for convenient (oral) drug delivery. Compositions are extracts made with hot water, alcoholic solvents (ethanol) and non-alocoholic organic solvents (ester, lipid, ethyl acetate, etc.).

Overall the botanical compositions show mostly cytostatic effect with very weak cytotoxic effects in the compositions of the invention. Histopathology of cells treated with the compositions of the invention indicates minimal retention of dead cancer cells which enhance recovery following cancer therapy.

### EXAMPLES

Without further elaboration, it is believed that one skilled in the art can, using the preceding description, utilize the present invention to its fullest extent. The following examples are illustrative only, and not limiting of the remainder of the disclosure in any way whatsoever.

The following combinations of extracts were used throughout the examples: *Ganoderma lucidum, Scutellaria barbata*, optionally *Scutellaria baicalensis* and *Salvia miltiorrhiza* extracts when combined in ratios that exhibit synergism are variously referred to as OMN54 or Aneustat throughout the specification.

In addition, the compositions of the invention may include, optionally, Panax Quinquefolium (Western ginseng), *Camellia sinensis* (green tea), and *Hippophae rhamnoides* (sea buckthorn).

One skilled in the art would appreciate that while the following examples are illustrative of the invention, any cell line may be used. For example, although not limiting, cells may be obtained from ATCC, Rockville, Md.

Experiments to determine efficacy of the novel compositions and formulations disclosed herein were carried out at the British Columbia Cancer Agency, Vancouver, Canada.

One skilled in the art would also appreciate that while the foregoing examples are illustrative of the invention, multiple prostate cancer in vivo models may be used. For example, CaP xenografts in mice may be utilized. Additionally, a Pten knockout mouse strain, in which heterozygous mice develop tumors of the uterus, prostate, thyroid, colon, and adrenal medulla, may be obtained from the Mouse Models of Human Cancers Consortium (Podsypanina K. et al., (1999) Proc. Nat'l Acad. Sci. USA 96: 1563-1568).

### Example 1: Methods for Preparation of Botanical Extracts

The compositions of the present invention may be administered as dried herbs. Botanical preparations contain phytochemicals some of which are soluble in aqueous media while others are relatively more soluble in organic (alcohol, ester, lipid) media. Different extraction methods were used and tested for the ability to extract effective ingredients from the herbs. Extraction methods include: Aqueous (hot water) extraction; Organic (lipid fraction) extraction; non-alcoholic organic (ethyl acetate) extraction; and alcohol (ethanol) Extraction.

Products are prepared from herbs or herb blends by extraction with solvent (hot water, 80% ethanol, or ethyl acetate) under reflux for 30-60 minutes, separated by filtration to obtain a filtrate, and concentrated (e.g., by evaporation) for further analysis. The filtrates were combined, diluted or concentrated prior to determination of activities. Various additives including, but not limited to, detergents, anti-oxidants and diluents were added to the extract.

### Example 2: Effects of Aneustat alone and docetaxel alone on Growth of LNCaP Prostate Cancer Cells

Both the botanical composition (Aneustat or OMN54) and Taxotere® (docetaxel) significantly inhibited growth of LNCaP (androgen-sensitive) human prostate cancer tumors SRCX *in vivo* (mice) (P<0.01). The effect of Aneustat on reducing tumor volume was comparable to that of standard chemotherapy with Taxotere® (which has significant toxicity).

LNCaP prostate cancer was treated for 3 weeks according to the following regimen.

| | **Aneustat** | **Taxotere® (docetaxel)** |
|---|---|---|
| **Delivery Route** | Oral gavage | IP |
| **Dose** | 1652 mg/kg body wt. | 10 mg/kg/QD |
| **Frequency** | QD x 21 | d=1,8,15 |

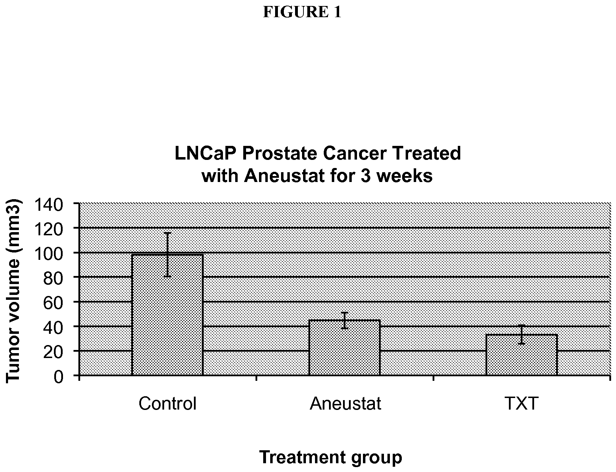

As seen in Figure 1, Aneustat and Taxotere® display comparable efficacies in tumor size reduction, with Taxotere® somewhat more effective.

### Example 3: Effects of Aneustat alone and docetaxel alone on Growth of DU145 Prostate Cancer Cells

Both the botanical composition (Aneustat or OMN54) or (Taxotere® (docetaxel) + Emcyt® (Pfizer)) significantly inhibited growth of DU145 (androgen-independent) human prostate cancer tumors SRCX in vivo (mice) (P<0.01). The effect of Aneustat on reducing tumor volume was comparable to that of standard chemotherapy with Emcyt (estramustine sodium phosphate) + Taxotere® (which has significant toxicity).

LNCaP prostate cancer was treated for 3 weeks according to the following regimen.

| | **Aneustat** | **Emcyt®** | **+** | **Taxotere®** |
|---|---|---|---|---|
| **Delivery Route** | Oral gavage | PO | | IP |
| **Dose** | 1652 mg/kg body wt. | 10mg/kg/QD | | 10 mg/kg/QD |
| **Frequency** | QD x 21 | d=1,2,3,4, 8, 9,10,11 | | d=1,8,15 |

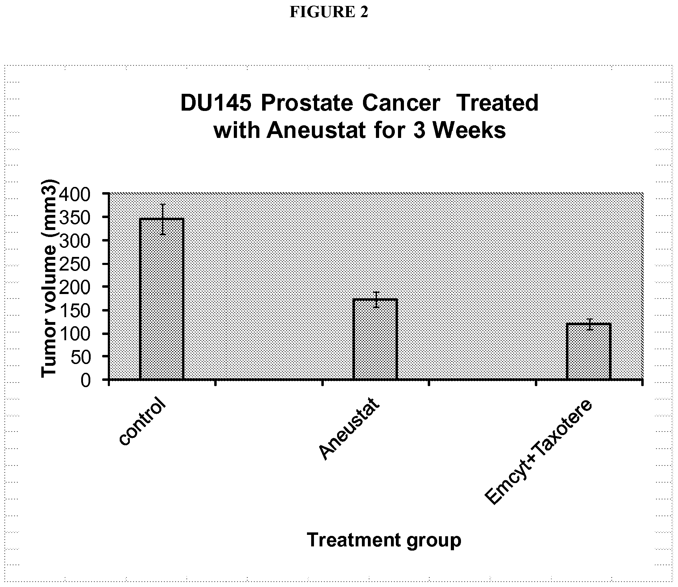

As seen in Figure 2, Aneustat and (Taxotere®+Emcyt®) display comparable efficacies in tumor size reduction, with (Taxotere®+Emcyt®) somewhat more effective.

### Example 5: Effect of OMN54 and docetaxel combination therapy on Prostate Cancer Tumor Shrinkage in vivo

After 3 weeks of treatment, the botanical composition (Aneustat or OMN54) in combination with Taxotere® (docetaxel) demonstrated synergy and tumor shrinkage when treating LTL-313 (androgen-dependent) human prostate cancer tumors SRCX in vivo (mice) (p=0).

LTL-313 prostate cancer was treated for 3 weeks according to the following regimen.

| | **Aneustat** | **Taxotere®** |
|---|---|---|
| **Delivery Route** | Oral gavage | IP |
| **Dose** | 1652 mg/kg body wt. | 5 mg/kg/QD |
| **Frequency** | QD x 21 | d=2, 9, 16 |

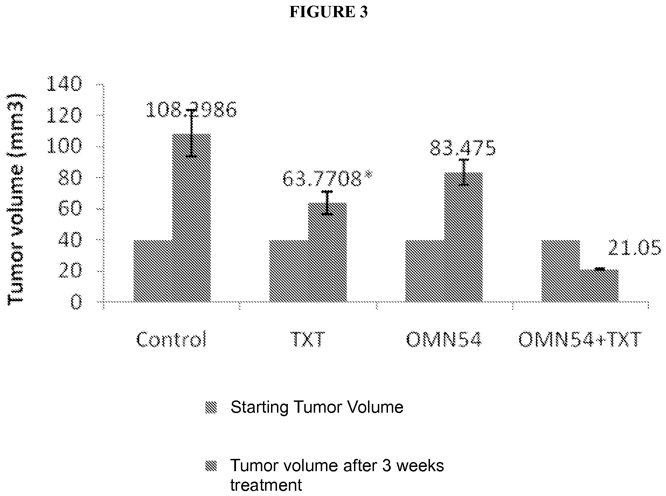

As seen in Figure 3, Aneustat and Taxotere® display synergistic efficacy in tumor size reduction.

### Example 6: Effect of OMN54 and docetaxel combination therapy on Prostate Cancer cell growth inhibition in vivo

After 3 weeks of treatment, the botanical composition (Aneustat or OMN54) in combination with Taxotere® (docetaxel), demonstrated significant growth inhibition when treating LTL-313 (androgen-dependent; adenocarcinoma) patient-derived prostate cancer tissue SRCX.

LTL-313H-13 xenografts were treated for 3 weeks according to the following regimen.

| **Treatment** | **Taxotere®** | **Combi-low** | | **Combi-med** | |
|---|---|---|---|---|---|
| | | **OMN54** | **Taxotere®** | **OMN54** | **Taxotere®** |
| **Dose** | 5 mg/kg/QD | 413/mg/kg | 5mg/kg/QD | 826/mg/kg | 5mg/kg/QD |
| **Delivery Route** | Oral gavage | Oral gavage | Oral gavage | Oral gavage | IP |
| **Schedule** | q7dx3 | q7dx3 | (Q1dx5)x3 | q7dx3 | (Q1dx5)x3 |

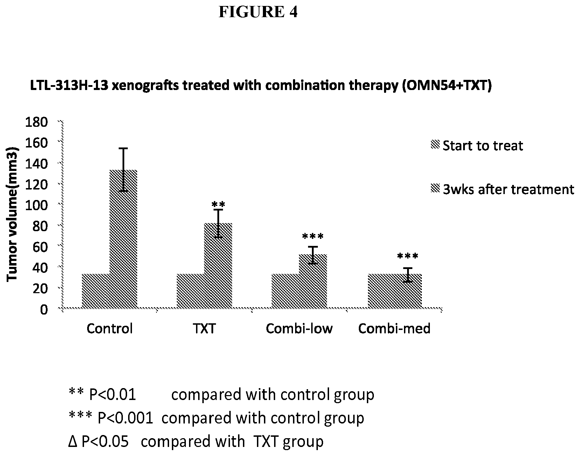

As seen in Figure 4, Aneustat and Taxotere® display synergistic efficacy in growth inhibition of prostate cancer cell xenografts in mice. Additionally, it is now demonstrated that the combination of these two agents appears to have greater therapeutic effect when treating LTL-313 (androgen-dependent; adenocarcinoma) patient-derived prostate cancer tissue SRCX as compared to Taxotere® alone.

### Example 7: Effect of OMN54 and docetaxel combination therapy on PSA levels

After 3 weeks of treatment, the botanical composition (Aneustat or OMN54) in combination with Taxotere® (docetaxel) demonstrated significant Prostate-Specific Antigen (PSA) level reduction-in a dose responsive manner-when treating LTL-313 (androgen-dependent; adenocarcinoma) patient-derived prostate cancer tissue SRCX.

LTL-313H-13 xenografts were treated for 3 weeks according to the following regimen.

| **Treatment** | **Taxotere®** | **Combi-low** | | **Combi-med** | |
|---|---|---|---|---|---|
| | | **OMN54** | **Taxotere®** | **OMN54** | **Taxotere®** |
| **Dose** | 5 mg/kg/QD | 413/mg/kg | 5mg/kg/QD | 826/mg/kg | 5mg/kg/QD |
| **Delivery Route** | Oral gavage | Oral gavage | Oral gavage | Oral gavage | IP |
| **Schedule** | q7dx3 | q7dx3 | (Q1dx5)x3 | q7dx3 | (Q1dx5)x3 |

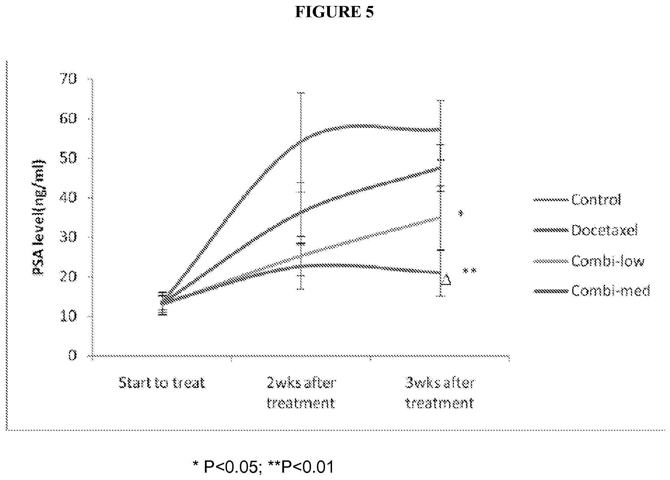

As seen in Figure 5, Aneustat and Taxotere® display synergistic efficacy in growth inhibition of prostate cancer cell xenografts in mice. Additionally, it is now demonstrated that the combination of these two agents appears to have greater therapeutic effect when treating LTL-313 (androgen-dependent; adenocarcinoma) patient-derived prostate cancer tissue SRCX as compared to Taxotere® alone.

### Example 8: Establishment of a human prostate cancer tissue xenograft/mouse model

A more predictive human tumor tissue xenograft model was used to assess efficacy of the botanical compositions. (Clin Cancer Research 2006: 12(13); 4043-4054) Human tumor grafts are better than cell line injections and human tumors grafted on kidney capsule have more immediate blood supply than subcutaneous grafts, thus more biological diversity is retained. The xenograft model more closely resembles clinical cancer than traditional *in vivo* models

Preclinical testing of prostate cancer therapeutics has been largely carried out using xenograft models in which human prostate cancer cell lines have been subcutaneously injected into immunodeficient mice. However, cancer cell xenografts may not accurately mimic the behavior of prostate tumors *in vivo.* In fact, cancer cell line xenograft models have a poor record of accurately predicting the clinical efficacy of anticancer agents. A novel xenograft model was established for a variety of pre-cancerous and cancerous human tissues, including prostate cancer tissue. Most importantly, the xenografts in the model retain the histological characteristics of the parental tissue. For selected types of cancer that the xenografts respond to therapy in a manner similar to that observed in patients. For example, prostate cancer tissue grown in SCID mice showed a dramatic response to androgen ablation therapy as regularly found in the clinic.

Xenografts of human prostate cancer cell line LTL-313 were grown in vivo. One of two tissue xenografts grew to the size of a walnut. Tumors are grafted to the renal site survive and retain their original histopathology and differentiation marker profile, even after serial passages. The prostate cancer tissue very rapidly grows in SCID mice with a doubling time about 5 days. Cytogenetic analyses show some abnormal chromosomes. Not only are there translocations, there are also deletions and duplication of chromosomal segments (Note: since each chromosome has its own display color, more than one color along the length of a chromosome indicates a translocation). The Spectral Karyotyping (SKY) analysis shows that the tissue of a LTL-313 cancer xenograft contained only a low number of karyotypic alterations, although the cancer is highly advanced.

### Example 9: Efficacy study on prostate cancer cell line (DU145)

Tumor xenografts from a prostate cell line DU145 were cut into 2 mm³ pieces and grafted into SCID/nod mice. Treatment was started at day 13 (mean volume = 15.6 mm³). The mice were divided into 3 equal groups for treatment with saline; *Ganoderma lucidum, Salvia miltiorrhiza*, and *Scutellaria barbata* at 3.3 IC₅₀; and estramustine sodium phosphate (EMCYT®) and docetaxel (E+D). The combination of *Ganoderma lucidum, Salvia miltiorrhiza*, and *Scutellaria barbata* showed a significant inhibitory effect comparable to the E+D regimen. OMN54 significantly inhibited growth of DU145 (androgen-independent) human prostate cancer tumors in vivo. Importantly, this effect on tumor volume was comparable to that of standard chemotherapy which is associated with significant toxicities to patients.

### Example 10: In vivo assay for activity in reducing prostate tumor size

6-week old male nude mice (BALB/c-nu/nu) are used for the experimental animal model. Animals are cut open from the abdomen and inoculated with the human prostate cancer cell lines- LTL-313 cells (2x 10⁶ cells/50µl/Hanks Buffered Saline Solution/mice) from back side of the prostate with a 30g needle. The cut abdomens are then sutured with 5-0 thread. After 2 weeks with regular feeding, blood is drawn from each animal for measuring the serum PSA value. The compositions of the invention are administered orally to the mice as follows; 3 groups of 6 mice received 43.65, 14.4, or 4.3 mg/animal/day for 21 days. Age-matched control mice are treated with saline for the same period.

Grafts are then harvested to determine the effect on tumor volume, histology, apoptosis index (Tunel assay) and proliferation index (proliferation marker Ki67 staining). The TUNEL assay detects apoptosis-induced DNA fragmentation through a quantitative fluorescence assay. Terminal deoxynucleotidyl transferase (TdT) catalyzes the incorporation of bromo-deoxyuridine (BrdU) residues into the fragmenting nuclear DNA at the 3'-hydroxyl ends by nicked end labeling. A TRITC-conjugated anti-BrdU antibody can then label the 3'-hydroxyl ends for detection.

## Claims

1. A botanical composition for use in a method of treating or reducing the severity of prostate cancer in a subject, the method comprising:
administering an effective amount of the composition,
wherein the composition comprises extracts in an organic medium of *Ganoderma lucidum, Salvia miltiorrhiza* and *Scutellaria barbata*, wherein each extract comprises from about 45 percent to about 50 percent w/w of *Ganoderma lucidum* and *Scutellaria barbata* and about 1 to about 3 percent w/w of *Salvia miltiorrhiza*, wherein each extract is a non-alcoholic extract, and wherein the botanical composition is effective for reducing a symptom associated with prostate cancer; and
co-administering an effective amount of docetaxel,
wherein the co-administration of the compound and the botanical composition achieves more effective therapy than administration of either agent alone, and results in reduced toxicity and side-effects compared to administration of an equivalent dosage of docetaxel alone.

2. The composition for use according to claim 1, wherein the prostate cancer symptom is selected from (a) a size of a prostate tumor; (b) a growth rate of prostate cancer tissue; and (c) a level of prostate specific antigen (PSA).

3. The composition for use according to claim 2, wherein the prostate tumor or the prostate cancer tissue is an androgen-dependent adenocarcinoma.

4. The composition for use according to claim 1, wherein the co-administration comprises daily administration of the botanical composition and periodic administration of docetaxel over a time period, optionally wherein the time period is 3 weeks.

5. The composition for use according to claim 1, wherein the extract is made with ethyl acetate ester.

6. The composition for use according to claim 1, wherein the botanical composition comprises 2% w/w of *Salvia miltiorrhizza* extract.

7. The composition for use according to claim 1, wherein the botanical composition comprises one or more of an emulsifier, a surfactant, an antioxidant and a diluent.

8. The composition for use according to claim 7, wherein the emulsifier is selected from one or more of fatty acids, polyoxyethylene glycerol esters of fatty acids, polooxylated castor oil, ethylene glycol esters, propylene glycol esters glyceryl esters of fatty acids, sorbitan esters, polyglyceryl esters, fatty alcohol ethoxylates, ethoxylated propoxylated block copolymers, polyethylene glycol esters of fatty acids, cremophores, glycerol monocaprylate/caprate, Cremophor EL, oleic acid, Labrasol, Gelucire, Capryol, Captex, Acconon, transcutol, and triacetin.

9. The composition for use according to claim 7, wherein the antioxidant is selected from ascorbic acid and alpha tocopherol.

10. The composition for use according to claim 7, wherein the diluent is soya oil.

11. The composition for use according to claim 1, wherein docetaxel is administered intravenously and the botanical composition is administered orally.

12. The composition for use according to claim 1, wherein the prostate cancer is selected from a castration-resistant prostate cancer and an androgen-sensitive prostate cancer.

13. The composition for use according to claim 1, wherein the docetaxel is administered in a sub-therapeutic dose.

14. The composition for use according to claim 1, wherein botanical composition comprises:
(a) an effective amount of Ganoderma lucidum extract selected from 45%, 46%, 46.5%, 47%, 47.5%, 48%, 48.5%, 49%, 49.5% and 50%;
(b) an effective amount of Scutellaria barbata extract selected from 45%, 46%, 46.5%, 47%, 47.5%, 48%, 48.5%, 49%, 49.5% and 50%; and
(c) an effective amount of Salvia miltiorrhiza extract selected from 1%, 1.5%, 2%, 2.5% and 3%.

15. The composition for use according to claim 1, wherein botanical composition is in a dosage form suitable for oral administration.

## Patentansprüche

1. Botanische Zusammensetzung zur Verwendung in einem Verfahren zum Behandeln oder Verringern der Schwere von Prostatakrebs in einem Subjekt, das Verfahren umfassend:
Verabreichen einer wirksamen Menge der Zusammensetzung,
wobei die Zusammensetzung Extrakte in einem organischen Medium aus Ganoderma lucidum, Salvia miltiorrhiza und Scutellaria barbata umfasst, wobei jeder Extrakt von ungefähr 45 bis ungefähr 50 Gew.-% Ganoderma lucidum und Scutellaria barbata und ungefähr 1 bis ungefähr 3 Gew.-% Salvia miltiorrhiza umfasst, wobei jeder Extrakt ein nicht alkoholischer Extrakt ist, und wobei die botanische Zusammensetzung zum Verringern eines mit Prostatakrebs verbundenen Symptoms wirksam ist; und
gleichzeitiges Verabreichen einer wirksamen Menge von Docetaxel,
wobei die gleichzeitige Verabreichung der Verbindung und der botanischen Zusammensetzung eine wirksamere Therapie als die alleinige Verabreichung eines der Mittel erreicht, und in verringerter Toxizität und Nebenwirkungen verglichen mit der Verabreichung einer äquivalenten Dosis von Docetaxel allein resultiert.

2. Zusammensetzung zur Verwendung nach Anspruch 1, wobei das Prostatakrebssymptom ausgewählt ist aus: (a) einer Größe eines Prostatatumors; (b) einer Wachstumsrate von Prostatakrebsgewebe; und (c) einer Höhe von Prostata-spezifischem Antigen (PSA).

3. Zusammensetzung zur Verwendung nach Anspruch 2, wobei der Prostatatumor oder das Prostatakrebsgewebe ein Androgen-abhängiges Adenokarzinom ist.

4. Zusammensetzung zur Verwendung nach Anspruch 1, wobei die gleichzeitige Verabreichung tägliche Verabreichung der botanischen Zusammensetzung und periodische Verabreichung von Docetaxel über einen Zeitraum umfasst, optional wobei der Zeitraum 3 Wochen ist.

5. Zusammensetzung zur Verwendung nach Anspruch 1, wobei der Extrakt mit Ethylacetatester hergestellt wird.

6. Zusammensetzung zur Verwendung nach Anspruch 1, wobei die botanische Zusammensetzung 2 Gew .-% Salvia miltiorrhizza-Extrakt umfasst.

7. Zusammensetzung zur Verwendung nach Anspruch 1, wobei die botanische Zusammensetzung einen oder mehrere von einem Emulgator, einem Tensid, einem Antioxidationsmittel und einem Verdünnungsmittel umfasst.

8. Zusammensetzung zur Verwendung nach Anspruch 7, wobei der Emulgator ausgewählt ist aus einer oder mehreren von Fettsäuren, Polyoxyethylenglycerinestern von Fettsäuren, polooxyliertem Rizinusöl, Ethylenglycolestern, Propylenglycolester-Glycerylestern von Fettsäuren, Sorbitanestern, Polyglycerylestern, Fettalkoholethoxylaten, ethoxylierten propoxylierten Blockcopolymeren, Polyethylenglykolestern von Fettsäuren, Cremophoren, Glycerinmonocaprylaten/capraten, Cremophor EL, Ölsäure, Labrasol, Gelucire, Capryol, Captex, Acconon, Transcutol und Triacetin.

9. Zusammensetzung zur Verwendung nach Anspruch 7, wobei das Antioxidationsmittel ausgewählt ist aus Ascorbinsäure und Alpha-tocopherol.

10. Zusammensetzung zur Verwendung nach Anspruch 7, wobei das Verdünnungsmittel Sojaöl ist.

11. Zusammensetzung zur Verwendung nach Anspruch 1, wobei Docetaxel intravenös verabreicht wird und die botanische Zusammensetzung oral verabreicht wird.

12. Zusammensetzung zur Verwendung nach Anspruch 1, wobei der Prostatakrebs aus einem kastrationsresistenten Prostatakrebs und einem Androgen-sensitiven Prostatakrebs ausgewählt ist.

13. Zusammensetzung zur Verwendung nach Anspruch 1, wobei das Docetaxel in einer subtherapeutischen Dosis verabreicht wird.

14. Zusammensetzung zur Verwendung nach Anspruch 1, wobei die botanische Zusammensetzung umfasst:
(a) eine wirksame Menge an Ganoderma lucidum-Extrakt, ausgewählt aus 45 %, 46 %, 46,5 %, 47 %, 47,5 %, 48 %, 48,5 %, 49 %, 49,5 % und 50 %;
(b) eine wirksame Menge an Scutellaria barbata-Extrakt, ausgewählt aus 45 %, 46 %, 46,5 %, 47 %, 47,5 %, 48 %, 48,5 %, 49 %, 49,5 % und 50 %; und
(c) eine wirksame Menge an Salvia miltiorrhiza-Extrakt, ausgewählt aus 1 %, 1,5 %, 2 %, 2,5 % und 3 %.

15. Zusammensetzung zur Verwendung nach Anspruch 1, wobei die botanische Zusammensetzung in einer Dosierungsform vorliegt, die zur oralen Verabreichung geeignet ist.

## Revendications

1. Composition botanique pour utilisation dans une méthode de traitement ou de réduction de la gravité du cancer de la prostate chez un sujet, la méthode comprenant :
l'administration d'une quantité efficace de la composition,
dans laquelle la composition comprend des extraits dans un milieu organique de *Ganoderma lucidum, Salvia miltiorrhiza* et *Scutellaria barbata,* dans laquelle chaque extrait comprend environ 45% à environ 50% en poids de *Ganoderma lucidum* et de *Scutellaria barbata* et environ 1 à environ 3% en poids de *Salvia miltiorrhiza*, chaque extrait étant un extrait non alcoolique et dans laquelle la composition botanique est efficace pour réduire un symptôme associé au cancer de la prostate ; et
la co-administration d'une quantité efficace de docétaxel,
dans laquelle la co-administration du composé et de la composition botanique constitue un traitement plus efficace que l'administration de l'un ou l'autre agent seul, et réduit la toxicité et les effets indésirables par rapport à l'administration d'une dose équivalente de docétaxel seul.

2. Composition pour utilisation selon la revendication 1, dans laquelle le symptôme de cancer de la prostate est choisi parmi (a) une taille d'une tumeur de la prostate ; (b) un taux de croissance du tissu du cancer de la prostate ; et (c) un niveau d'antigène prostatique spécifique de (APS).

3. Composition pour utilisation selon la revendication 2, dans laquelle la tumeur de la prostate ou le tissu de cancer de la prostate est un adénocarcinome androgéno-dépendant.

4. Composition pour utilisation selon la revendication 1, dans laquelle la co-administration comprend une administration quotidienne de la composition botanique et une administration périodique de docétaxel sur une période de temps, la période de temps étant éventuellement de 3 semaines.

5. Composition pour utilisation selon la revendication 1, dans laquelle l'extrait est préparé avec un ester d'acétate d'éthyle.

6. Composition pour utilisation selon la revendication 1, dans laquelle la composition botanique comprend 2% en poids d'extrait de *Salvia miltiorrhizza*.

7. Composition pour utilisation selon la revendication 1, dans laquelle la composition botanique comprend un ou plusieurs parmi un émulsifiant, un tensioactif, un antioxydant et un diluant.

8. Composition pour utilisation selon la revendication 7, dans laquelle l'émulsifiant est choisi parmi un ou plusieurs acides gras, esters de polyoxyéthylèneglycérol d'acides gras, l'huile de ricin polooxylée, esters d'éthylèneglycol, esters de propylèneglycol, esters de glycéryle d'acides gras, esters de sorbitane, esters de polyglycérol, éthoxylates d'alcools gras, copolymères séquencés d'éthoxylés propoxylés, esters de polyéthylèneglycol d'acides gras, crémophores, monocaprylate/caprate de glycérol, Crémophor EL, acide oléique, Labrasol, Gelucire, Capryol, Captex, Acconon, transcutol et triacétine.

9. Composition pour utilisation selon la revendication 7, dans laquelle l'antioxydant est choisi parmi l'acide ascorbique et l'alpha-tocophérol.

10. Composition pour utilisation selon la revendication 7, dans laquelle le diluant est de l'huile de soja.

11. Composition pour utilisation selon la revendication 1, dans laquelle le docétaxel est administré par voie intraveineuse et la composition botanique est administrée par voie orale.

12. Composition pour utilisation selon la revendication 1, dans laquelle le cancer de la prostate est choisi parmi un cancer de la prostate résistant à la castration et un cancer de la prostate sensible aux androgènes.

13. Composition pour utilisation selon la revendication 1, dans laquelle le docétaxel est administré à un dosage sous-thérapeutique.

14. Composition pour utilisation selon la revendication 1, dans laquelle la composition botanique comprend :
(a) une quantité efficace d'extrait de Ganoderma lucidum choisie parmi 45%, 46%, 46,5%, 47%, 47,5%, 48%, 48,5%, 49% %, 49,5% et 50% ;
(b) une quantité efficace d'extrait de Scutellaria barbata choisie parmi 45%, 46%, 46,5%, 47%, 47,5%, 48%, 48,5%, 49%, 49,5% et 50%; et
(c) une quantité efficace d'extrait de Salvia miltiorrhiza choisie parmi 1%, 1,5%, 2%, 2,5% et 3%.

15. Composition pour utilisation selon la revendication 1, dans laquelle la composition botanique est sous une forme posologique appropriée pour une administration orale.
